# EUROPEAN PATENT SPECIFICATION

(11) **EP 4 356 957 B1**
(45) Date of publication and mention of the grant of the patent: **04.11.2026**
(21) Application number: 23200259.2
(22) Date of filing: 27.09.2023
(51) Int. Cl.: A61N 1/39, A61B 5/00, A61B 5/318, A61B 5/0205, A61B 5/145

(54) **DEVICE FOR DEFIBRILLATION AND MONITORING, MONITORING COMPONENT AND COMPONENT FOR DEFIBRILLATION AND MONITORING**
VORRICHTUNG ZUR DEFIBRILLATION UND ÜBERWACHUNG, ÜBERWACHUNGSKOMPONENTE UND KOMPONENTE ZUR DEFIBRILLATION UND ÜBERWACHUNG
DISPOSITIF DE DÉFIBRILLATION ET DE SURVEILLANCE, COMPOSANT DE SURVEILLANCE ET COMPOSANT DE DÉFIBRILLATION ET DE SURVEILLANCE

(30) Priority: 27.09.2022 CN 202211185685; 27.09.2022 CN 202211186252
(43) Date of publication of application: 24.04.2024
(73) Proprietor: Shenzhen Mindray Bio-Medical Electronics Co., Ltd., Shenzhen, Guangdong 518057 (CN)
(72) Inventor: HE, Lijuan, Shenzhen, 518057 (CN); ZOU, Huan, Shenzhen, 518057 (CN); CHEN, Dabing, Shenzhen, 518057 (CN); JIANG, Jianfeng, Shenzhen, 518057 (CN); ZHANG, Peng, Shenzhen, 518057 (CN)
(74) Representative: KIPA AB

(56) References cited:
- US-A1- 2017 203 112
- US-A1- 2018 036 544
- US-A1- 2019 117 989
- US-A1- 2020 305 805
- US-A1- 2022 077 633

## Description

### TECHNICAL FIELD

The disclosure relates to the technical field of medical device, and more particularly to a device for defibrillation and monitoring, a monitoring component and component for defibrillation and monitoring.

### BACKGROUND

Defibrillation and vital sign monitoring are especially important operations in medical situations. For example, in prehospital emergency situations, first aiders need to carry many emergency device to the scene, including a vital sign monitoring device (referred to as monitoring device) and a defibrillation device.

In some products, the monitoring device and the defibrillation device are independent devices, occupying a significant amount of space in medical facilities. Moreover, when medical staff need to go out for medical assistance, they often need to carry both monitoring device and defibrillation device, causing great inconvenience to them.

**In** other products, modular design is employed, in which the display module, monitoring module, and defibrillation module are arranged to be three modules that can be combined into a single integral structure. However, due to the fact that both the monitoring module and defibrillation module need to be used together with the display module, it is still inconvenient to carry them when a monitoring operation or a defibrillation operation is required independently.

US20200305805A1 discloses a medical device integrated with portable display and functionality.

### SUMMARY

This disclosure is as defined in the appended claims. The device for defibrillation and monitoring according to the above embodiment includes a host and a monitoring apparatus, which can be assembled and disassembled through a detachably connection. When both defibrillation and monitoring operations need to be carried out outside, the host and monitoring apparatus can be combined into a whole for portability. Meanwhile, this host can independently perform defibrillation operations and display defibrillation information at least. Therefore, when it is necessary to go out for defibrillation operation alone, only the host can be carried to medical assistance facilities. This monitoring apparatus can independently implement monitoring functions. When it is necessary to go out for implementing separate monitoring operations, just the monitoring apparatus can be carried to the medical assistance facilities. Therefore, the users can flexibly choose the devices they carry according to their requirements, improving the convenience of use. At the same time, the monitoring apparatus is also provided with a first storage member, which is configured to store monitoring accessories (such as sensors and connecting cables), thereby avoiding disorderly placement of monitoring accessories that occupy already limited space and influence on the work of the user.

The device for defibrillation and monitoring according to the above embodiment includes a display apparatus and an apparatus for defibrillation and monitoring, which can be assembled and disassembled through a detachably connection. The apparatus for defibrillation and monitoring includes an integrated machine for defibrillation and monitoring, which can independently complete monitoring and defibrillation operations. When a larger display window is needed for displaying, the display apparatus and the apparatus for defibrillation and monitoring can be combined into a whole and used together to display defibrillation information and monitoring information through the display apparatus. But when it is necessary to reduce the weight and volume of the device, only the apparatus for defibrillation and monitoring can be carried to the medical assistance facilities. Therefore, users can flexibly choose the devices they carry according to their requirements, improving the convenience of use. At the same time, the apparatus for defibrillation and monitoring is also provided with a first storage member, which is configured to store defibrillation and/or monitoring accessories (such as sensors and connecting cables for monitoring, electrodes, electrode sheets for defibrillation, etc.), thereby avoiding the disorderly placement of accessories that occupy already limited space and influence on the work of the user.

The monitoring component according to the above embodiment includes a monitoring module and a second storage member, which is configured to store the monitoring module, that is, the monitoring module can be placed inside the second storage member. Moreover, the second storage member also has a second docking structure, through which the second storage member, along with the monitoring module, can be assembled with other apparatus, such as a host, so that the monitoring module can form an assembly structure with other apparatus, which assembly structure is capable of being disassembled.

The monitoring component according to the above embodiment includes a monitoring module and a first storage member, wherein the first storage member is detachably connected with the monitoring apparatus, or the first storage member is affixed to the monitoring apparatus, or the first storage member and the monitoring apparatus are integrally formed. The first storage member is configured to store monitoring accessories (such as sensors and connecting cables), thereby avoiding disorderly placement of monitoring accessories that occupy already limited space and influence on the work of the user.

The component for defibrillation and monitoring according to the above embodiment includes an integrated machine for defibrillation and monitoring and a second storage member, which is configured to store the integrated machine for defibrillation and monitoring, that is, the integrated machine for defibrillation and monitoring can be placed inside the second storage member. Moreover, the second storage member is also provided with a second docking structure, through which the second storage member, along with the integrated machine for defibrillation and monitoring, can be assembled with other apparatus, such as the display apparatus mentioned above, so that the integrated machine for defibrillation and monitoring can form an assembly structure with other apparatus, which assembly structure is capable of being disassembled.

The component for defibrillation and monitoring according to the above embodiment includes an integrated machine for defibrillation and monitoring and a first storage member, wherein the first storage member is detachably connected with the integrated machine for defibrillation and monitoring, or the first storage member is affixed to the integrated machine for defibrillation and monitoring, or the first storage member and the integrated machine for defibrillation and monitoring are integrally formed. The first storage member is configured to store defibrillation and/or monitoring accessories (such as sensors and connecting cables for monitoring, electrodes, electrode sheets for defibrillation, etc.), thereby avoiding the disorderly placement of accessories that occupy already limited space and influence on the work of the user.

### BRIEF DESCRIPTION OF THE DRAWINGS

FIG. 1 is an assembly diagram of a host and a monitoring apparatus in an embodiment of this disclosure, which assembly is capable of being disassembled.
FIG. 2 is a lateral assembly diagram of a host and a monitoring apparatus in an embodiment of this disclosure, which assembly is capable of being disassembled.
FIGS. 3 and 4 are diagrams of an assembly area for extension module formed by three surfaces of a host housing in an embodiment of this disclosure.
FIG. 5 is a structural diagram of an assembly area for extension module at a host in an embodiment of this disclosure.
FIG. 6 is a diagram showing a process for disassembling a monitoring module from a second storage member in an embodiment of this disclosure, in which embodiment a first storage member is provided at a monitoring apparatus.
FIG. 7 is a diagram of a cable layout at a monitoring module when the monitoring module is arranged at a second storage member in an embodiment of this disclosure.
FIG. 8 is a structural diagram of a monitoring apparatus and a first storage member in another embodiment of this disclosure.
FIGS. 9 and 10 are structural diagrams of a monitoring apparatus and a first storage member in another more detailed embodiment of this disclosure.
FIG. 11 is a diagram of a third storage member, which is arranged at a rear side of a monitoring apparatus, in an embodiment of this disclosure.
FIGS. 12-13 are diagrams of a third storage member, which is arranged at a rear side of a monitoring apparatus and assembled with a hose together with the monitoring apparatus, in an embodiment of this disclosure.
FIG. 14 is a diagram of a third storage member, which is arranged at a rear side of a host in an embodiment of this disclosure.
FIG. 15 is a diagram of a third storage member, which is located in front of an assembly area for extension module in an embodiment of this disclosure.
FIG. 16 is a diagram of a third storage member, which is arranged at a rear side of a host in another embodiment of this disclosure.
FIG. 17 is a diagram of the structure shown in FIG. 16 assembled with a monitoring apparatus and a first storage member.
FIG. 18 is a diagram of an embodiment of this disclosure, in which a bottom of a front side of a host is provided with an assembly chamber for electrode sheet.
FIG. 19 is a diagram showing storage of electrode sheet and cable in an embodiment of this disclosure, in which a bottom of a front side of a host is provided with an assembly chamber for electrode sheet, and left and right sides of the host are provided with a third storage member.
FIG. 20 is a diagram of an embodiment of this disclosure, in which a side surface, which is located in front of an assembly area for extension module, is provided with an assembly chamber for electrode sheet, and a host is provided with a third storage member at its rear side.

### DESCRIPTION OF THE PREFERRED EMBODIMENTS

This disclosure is further explained in detail through specific implementation methods combined with the accompanying drawings. Similar components in different implementations use associated similar component reference numbers. In the following implementation methods, many details are described to enable this disclosure to be better understood. However, those skilled in this field can effortlessly recognize that some of these features can be omitted in different situations or replaced by other components, materials, or methods. **In** some cases, some operations related to this disclosure are not shown or described, so as to avoid the core part of this disclosure being overwhelmed by too many descriptions. For those skilled in the art, a detailed description of these related operations is not necessary. They can fully understand the relevant operations based on the description in this disclosure and general technical knowledge in the art.

**In** addition, the features, operations, or characteristics described in this disclosure can be combined in any appropriate manner to form various implementation methods. At the same time, the steps or actions in the method description can also be sequentially swapped or adjusted in a manner that is readily apparent to those skilled in the art. Therefore, the various sequences in the specification and accompanying drawings are only for the purpose of clearly describing a particular embodiment and do not necessarily imply a necessary sequence, unless otherwise stated that one of the sequences must be followed.

The numbering of components in this disclosure, such as "first", "second", etc., is only used to distinguish the described objects and does not have any order or technical meaning. The terms "connection" and "linkage" mentioned in this disclosure, unless otherwise specified, include both direct and indirect connections (linkages).

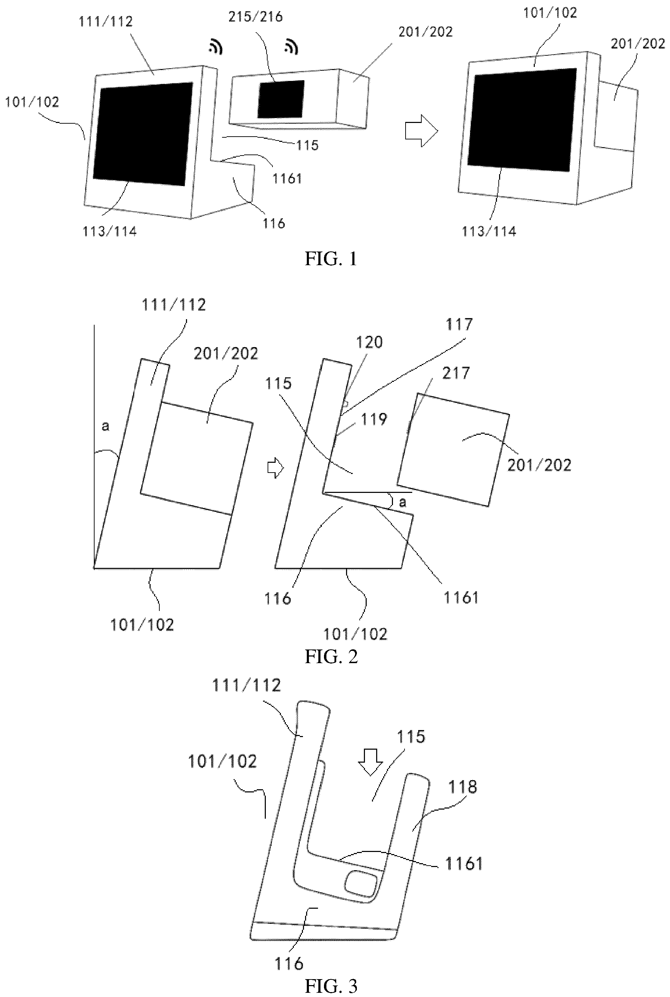

In order to adapt to different medical scenarios, especially when medical staff need to go out to medical assistance facilities for medical assistance, and to make it more convenient for medical staff to carry corresponding medical device according to their requirements, some embodiments of this disclosure provide a device for defibrillation and monitoring, referring to FIGS. 1 and 2. The device for defibrillation and monitoring includes a host 101 and a monitoring apparatus 201. The host 101 and the monitoring apparatus 201 can be assembled and disassembled through a detachably connection. When both defibrillation and monitoring operations need to be carried out outside, the host 101 and the monitoring apparatus 201 can be combined into a whole for portability. The host 101 can independently perform defibrillation operations and can be used to display defibrillation information at least. Therefore, when it is necessary to go out and perform defibrillation operations separately, only the host 101 can be carried to medical assistance facilities. The monitoring apparatus 201 can independently implement monitoring functions. When it is necessary to go out and separately perform monitoring operations, only the monitoring apparatus 201 can be carried to the medical assistance facilities. Therefore, users can flexibly choose the devices they carry according to their requirements, improving the convenience of use.

In some embodiments, the host 101 includes components, such as a host housing 111, a host control unit, at least a host display unit 113 for displaying defibrillation information, a high-voltage capacitor, a charging circuit, a discharging circuit, and a host information transmission unit for information transmission. The host control unit, high-voltage capacitor, charging circuit, and discharging circuit are all stored inside the host housing 111, so they are not shown in the attached figure.

The host housing 111 is a frame structure of the host 101, which is provided with a first accommodating chamber. The host control unit, the high-voltage capacitor, the charging circuit, and the discharging circuit are all located inside the first accommodating chamber, forming an integral module. The host housing 111 can usually be a housing of the host 101, but in some embodiments, the housing can also be at least partially unexposed. That is, other structures, such as decorative housings or other structures for decoration, can be provided outside the host housing 111.

The high-voltage capacitor is configured to store electrical energy for defibrillation, the charging circuit is configured to charge electrical energy, and the discharging circuit is configured to discharge electrical energy to the patient. The host control unit controls the charging and discharging circuits to complete a charging and discharging process of the high-voltage capacitor, and generates corresponding defibrillation information to implement defibrillation function, which can include manual defibrillation and automatic defibrillation. The specific defibrillation principle can refer to existing technologies and is not introduced too much here.

In an embodiment, the host 101 may include a detection circuit for thoracic bioimpedance which is configured to acquire detection data of thoracic bioimpedance. The host control unit receives detection data of thoracic bioimpedance and controls the charging and discharging circuits to complete the charging and discharging process of the high-voltage capacitor.

Please refer to FIGS. 1 and 2. In some embodiments, the host display unit 113 is arranged at a front side of the host housing 111. The front side is usually a side facing the user when the host 101 is in normal use. Of course, in other embodiments, the host display unit 113 can also be arranged at other sides of the host 101, such as a top surface or other side surfaces of the host 101. The host display unit 113 can use a pure display screen component for information display only. At this time, the host 101 can be configured with physical or touch buttons for instruction input and operation. Of course, in other embodiments, the host display unit 113 can also use a touch screen component to simultaneously display information and input instructions.

The host display unit 113 can be configured to display defibrillation information separately (such as defibrillation energy, etc.). In addition, when the host 101 is in communicative connection with the monitoring apparatus 20, the host display unit 113 can also be configured to display monitoring information (such as electrocardiogram, respiration, blood oxygen, etc.). The host 101 can be in wired and/or wireless communication connected with the monitoring apparatus 201 through the host information transmission unit to implement unidirectional information transmission or bidirectional information transmission. The unidirectional information transmission can be unidirectional information transmission from the monitoring apparatus 201 to the host 101 for transmitting monitoring information to the host 101 or displaying monitoring information on the host display unit 113. The unidirectional information transmission can also be unidirectional information transmission from the host 101 to the monitoring apparatus 201. The bidirectional information transmission involves information interaction between the host 101 and the monitoring apparatus 201. In some embodiments, the host 101 can also be in communicative connection with other medical devices (such as ultrasound devices, etc.) to acquire other medical information, which is correspondingly displayed at the host display unit 113.

The monitoring apparatus 201 can implement monitoring function regardless of whether it is assembled with the assembly structure or not, that is, the monitoring apparatus 201 can be disassembled from the host 101 and independently complete the monitoring function, or can be assembled with the host 101 to cooperate with the host 101 to implement the monitoring function.

Compared to the prior art, in which the display module, the monitoring module, and the defibrillation module are configured as three modules which are capable of being combined, the reduced number of modules in this device makes it more convenient for device installation and fixation, as well as for carrying devices to the scene for rescue. This allows the user to strike a balance between flexibility and device management convenience. The independently usable monitoring apparatus 201 has a separate display unit of monitoring apparatus 215, which can display information without depending on the host 101, thus greatly improving portability and satisfying the requirements of most scenarios that only require monitoring, and more accurately adapting to prehospital emergency scenes.

Moreover, the host 101 has defibrillation function, such that, like the monitoring apparatus 201, the defibrillation function can be used independently after separation, making it more flexible to adapt to different emergency scenarios. The monitoring apparatus 201 does not require to integrate the defibrillation function, so that it has a lighter volume and weight, and better portability.

The assembly and disassembly method of the host 101 and the monitoring apparatus 201 can adopt an up-down assembly, a left-right assembly, and a front-rear assembly. Wherein, a rear side refers to a side of the host 101 that is opposite to the front side, a left side refers to a side which corresponds to the left hand when the user faces the front side of the host 101, and a right side refers to a side which corresponds to the right hand when the user faces the front side of the host 101.

In some embodiments, in order to accommodate the monitoring apparatus 201, the host housing 111 has an assembly area for extension module 115, which has a shape that can accommodate at least part of the monitoring apparatus 201. The assembly area for extension module 115 can be either a chamber structure, an L-shaped open structure, an assembly platform, or other shapes. In some embodiments, the assembly area for extension module 115 can also accommodate other functional modules, such as ultrasound modules, respiratory modules, etc.

In some embodiments, when the monitoring apparatus 201 is located within the assembly area for extension module 115, and assembled with the assembly structure, with the host display unit 113 as a reference, the monitoring apparatus 201 can be arranged at at least one side of left, right, rear, upper, and lower sides of the host display unit 113. For example, in the embodiments shown in FIGS. 1-5, the monitoring apparatus 201 is located at the rear side of the host display unit 113. In the embodiments shown in FIGS. 1-2, the front-rear assembly of the host 101 and the monitoring apparatus 201 ensures a balanced layout of the entire device, which can reduce the integral height, the left and right width of the device.

Of course, in other embodiments, the monitoring apparatus 201 can also be arranged at the front side of the host display unit 113.

In the disassembly direction, in some embodiments, the monitoring apparatus 201 is capable of being placed into and/or taken out of the assembly area for extension module, from at least one of: a rear side, a left side, a right side, an upper side, and a lower side of the assembly area for extension module 115. For example, in some embodiments, at least one of: the rear side, the left side, the right side, the upper side, and the lower side of the assembly area for extension module 115 is open, such that the monitoring apparatus 201 is capable of being placed into and/or taken out of the assembly area for extension module 115 from the at least one open side. The at least one open side is exposed or provided with a barrier structure which is capable of being opened and closed. The barrier structure can adopt but is not limited to shielding covers, etc.

Please refer to FIGS. 1 and 2. In some embodiments, the monitoring apparatus 201 is capable of being taken out of the assembly area for extension module 115, from the rear side, the left side, the right side, or the upper side of the assembly area for extension module 115. Please refer to Figure 3. In some embodiments, the monitoring apparatus 201 is capable of being taken out of the assembly area for extension module 115, from the upper side, the left side, or the right side of the assembly area for extension module 115.

Furthermore, please refer to FIGS. 1-5. In some embodiments, at least two surfaces of the host housing 111 define the assembly area for extension module 115. When the monitoring apparatus 201 is assembled inside the assembly structure, at least a portion of the monitoring apparatus 201 is stored inside the assembly area for extension module 115, that is, the monitoring apparatus 201 can be fully or partially stored inside the assembly area for extension module 115.

Please refer to FIGS.1, 2, and 5. In some embodiments, the host housing 111 is provided with a support platform 116. The support platform 116 includes a support surface 1161, wherein the at least two surfaces of the host housing, which define the assembly area for extension module 115, include a support surface 1611. The assembly area for extension module 115 is arranged at an upper side of the support platform 116, and the support surface 1161 is configured to support the monitoring apparatus 201.

Specifically, please refer to FIG. 2. In some embodiments, the at least two surfaces of the host housing, which define the assembly area for extension module 115, includes the support surface 1161 and a side surface 1171 which is located in front of the assembly area for extension module 115. The support surface 1161 and the side surface 1171 enclose an L-shaped open structure, which is more convenient for the assembly and disassembly of the monitoring module. The monitoring apparatus 201 can be stored and placed into the open structure. The monitoring apparatus 201 can be placed at and/or taken out of the support platform 116 from at least one of: the rear, left, right, and upper sides of the assembly area for extension module 115.

The at least two surfaces of the host housing, which define the assembly area for extension module 115, further include other surfaces. For example, referring to FIGS. 3-5, in an embodiment, a rear end of the support platform 116 may also have a rear side part 118 which protrudes upwardly. A front side surface 1181 of the rear side part 118 which faces forward, together with the support surface 1161 and the side surface 1171, forms the assembly area for extension module 115. The monitoring apparatus 201 can be accommodated within the assembly area for extension module 115. The monitoring apparatus 201 can be taken out from the upper side, left side, and/or right side of the assembly area for extension module 115. In addition, said other surfaces can also be arranged at the left side or right side of the assembly area for extension module 115.

Furthermore, please refer to FIGS. 1-5. In some embodiments, the support platform 116 is arranged at the rear side of the host display unit 113, which can prevent the monitoring apparatus 201 from occupying the space at the front side of the host display unit 113, ensuring that the host display unit 113 has sufficient display area, and achieving a larger area of display.

Further, when the host display unit 113 is arranged at the front side of the host 101, please refer to FIG. 2 for more convenient viewing. In an embodiment, when the device for defibrillation and monitoring is placed horizontally, a first angle a is formed between the host display unit 113 and a vertical direction, which angle makes a display surface of the host display unit 113 to form an elevation angle, so that the user can view the display surface from high to low.

In addition, please refer to FIG. 2. In an embodiment, the support surface 1161 is inclined downward in a front-rear direction for the first angle a. The first angle a enables the monitoring apparatus 201 to be arranged backward for an elevation angle, which is more convenient for the assembly and disassembly of the monitoring apparatus 201. Of course, the downward inclination angle of the support surface 1161 may have a small deviation from the first angle a, such as a deviation within 5 degrees, as long as it does not affect the assembly of the monitoring apparatus 201.

Furthermore, in order to implement communicative connection between the host 101 and the monitoring apparatus 201, the host information transmission unit may choose a communication unit with a physical docking unit and/or a wireless communication unit (such as WIFI, Bluetooth, cellular data, etc.) that implements wireless communication. Correspondingly, the monitoring apparatus 201 has an information transmission unit that is consistent with the host information transmission unit. The information transmission unit of the monitoring module can choose a communication unit with a physical docking unit and/or a wireless communication unit that implements wireless communication. The host 101 and the monitoring apparatus 201 can not only use the host information transmission unit and the information transmission unit of the monitoring module to implement the communicative connection, but also can use the host information transmission unit and the information transmission unit of the monitoring module to implement the communicative connection with other apparatus, such as implementing unidirectional data transmission or data interaction with hospital devices. After the host 101 and the monitoring apparatus 201 are separated, if information transmission is to be continued, a wireless communication can be selected for implementing such information transmission.

In addition, the host information transmission unit can also be used as a power supply, such as for supplying power to the monitoring apparatus 201 through the host 101. Optionally, the monitoring apparatus 201 can supply power to the host 101. When the host information transmission unit and/or the information transmission unit of the monitoring module are used as power supply components, they can be realized through wireless charging or wired connection. Of course, additional charging circuits can also be provided at the host 101 and monitoring apparatus 201 to implement charging.

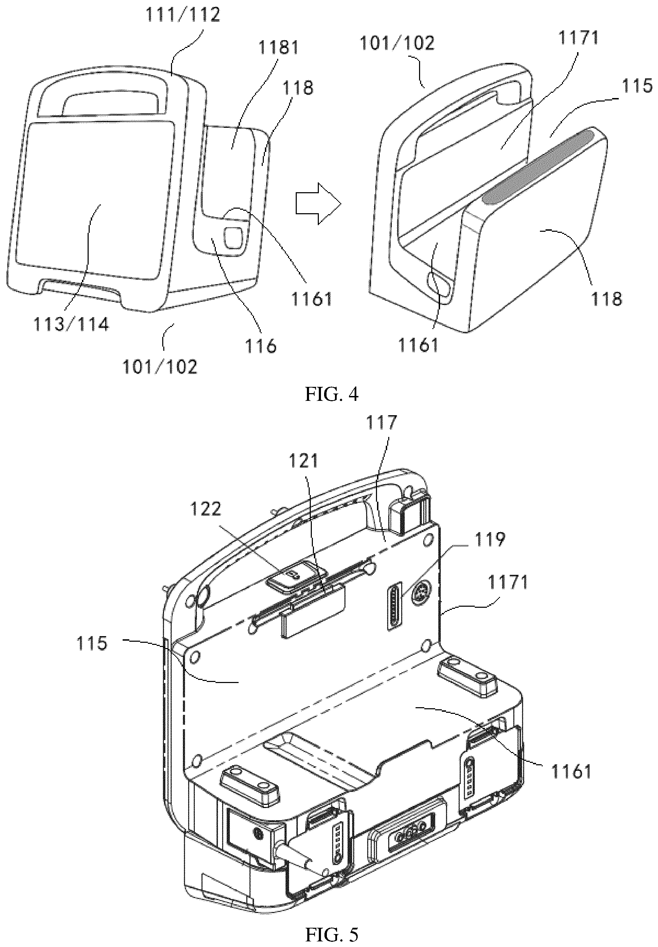

Please refer to FIGS. 2 and 5. In an embodiment, the host information transmission unit includes a docking part 119 which is connected with the monitoring apparatus 201, wherein the docking part is arranged at the side surface 1171 which is located in front of the assembly area for extension module 115. The docking part 119 can be a metal contact, plug interface, or other type of physical docking part 119. Correspondingly, the information transmission unit of the monitoring module also has a second docking part 217 which is adapted to the docking part 119. When the monitoring apparatus 201 is assembled into the assembly area for extension module 115, the second docking part 217 comes into contact with the docking part 119 to implement data communication. For example, in the embodiment shown in FIG. 2, the docking part 119 and the second docking part 217 are metal contacts. In the embodiment shown in FIG. 5, the docking part 119 is a plug interface, and in this case, the second docking section 217 can be a plug interface of the same specification.

In addition, the docking part 119 can also be arranged at other surfaces of the assembly area for extension module 115, such as the bottom surface of the assembly area for extension module 115 (such as the support surface 1161), or on the basis of the embodiments shown in FIGS. 3 and 4, the docking part 119 can be arranged at the front side surface 1181 of the rear side part 118.

In order to prevent the monitoring module from falling off the host 101 and enable the assembly between the host 101 and the monitoring apparatus 201 to be capable of being disassembled, the host housing 111 is provided with an assembly structure 120, and the monitoring apparatus 201 can be assembled with the assembly structure 120 and capable of being disassembled from the assembly structure 120. Wherein, the assembly structure 120 may include but is not limited to a clamping structure, a magnetic structure, a structure that is capable of being detachably adhered, a screwing structure, a tight fitting structure, etc.

In an exemplary structure, please refer to FIG. 5, the assembly structure 120 includes a locking member 121 and an unlocking member 122. The locking member 121 protrudes towards the assembly area for extension module 115. In the figure, the locking member 121 is arranged upwardly. When the monitoring module is assembled from top to bottom into the assembly area for extension module 115, it can form a snap-fit structure with the locking member 121. The unlocking member 122 and the locking member 121 form a linkage structure. By controlling the movement of the unlocking member 122, the locking member 121 can be driven to move, which enables the user to drive the locking member 121 to move in a direction away from the monitoring module through the unlocking member 122, so as to unlock a locking state between the locking member 121 and the monitoring module. If the unlocking member 122 drives the locking member 121 to move downwards to be disassembled from the snap-fit structure on the monitoring module, the monitoring module can be conveniently taken away at this time.

The locking member 121 can be arranged at at least one surface which forms the assembly area for extension module 115, for example, in FIG. 5, the locking member 121 is arranged at one side surface which faces the assembly area for extension module 115, such as side surface 1171. In addition, the locking member 121 can also be arranged at the bottom surface or other surfaces.

The unlocking member 122 and the locking member 121 can be arranged at the same surface or two adjacent surfaces respectively. For example, in FIG. 5, the unlocking member 122 and the locking member 121 are arranged at two adjacent surfaces of a side part 117. Wherein, the unlocking member 122 can be arranged at a top surface of the side part 117, and the locking member 121 is arranged at the side surface 1171 of the side part 117, which not only facilitates the locking member 121 and the monitoring module to form the snap-fit structure, but also facilitates the user to trigger the unlocking member 122 for unlocking. In addition, the unlocking member 122 and the locking member 121 can also be arranged at the same surface or adjacent surfaces of the support platform 116, for example, both are arranged at the support surface 1161 of the support platform 116. The unlocking member 122 and locking member 121 can also be arranged at adjacent surfaces of the support platform 116 and the side part 117. Of course, in addition to being arranged at two adjacent surfaces, the unlocking member 122 and the locking member 121 can also be arranged at different surfaces inside the host 101 that are completely not adjacent.

Of course, the above is only embodiment of the locking member 121, the unlocking member 122, or the entire docking structure. In other embodiments, other assembly structures that can implement locking and unlocking can also be used.

Furthermore, the monitoring apparatus 201, as an integral module which is independent relative to the host 101, can be used alone or assembled with the host 101 for use. Usually, the monitoring apparatus 201 includes monitoring accessories, which can include sensors and cables for at least one parameter of electrocardiogram, blood oxygen, non-invasive blood pressure, invasive blood pressure, body temperature, and exhaled gas. Often, these monitoring accessories are not specifically stored, or are stored inside a separate box. When not specifically stored, these accessories are placed in a disorderly manner, which occupies the already limited medical space (such as the space inside an ambulance). When stored in a separate box, it is not convenient to carry the monitoring accessories and monitoring apparatus 201, causing inconvenience to the user.

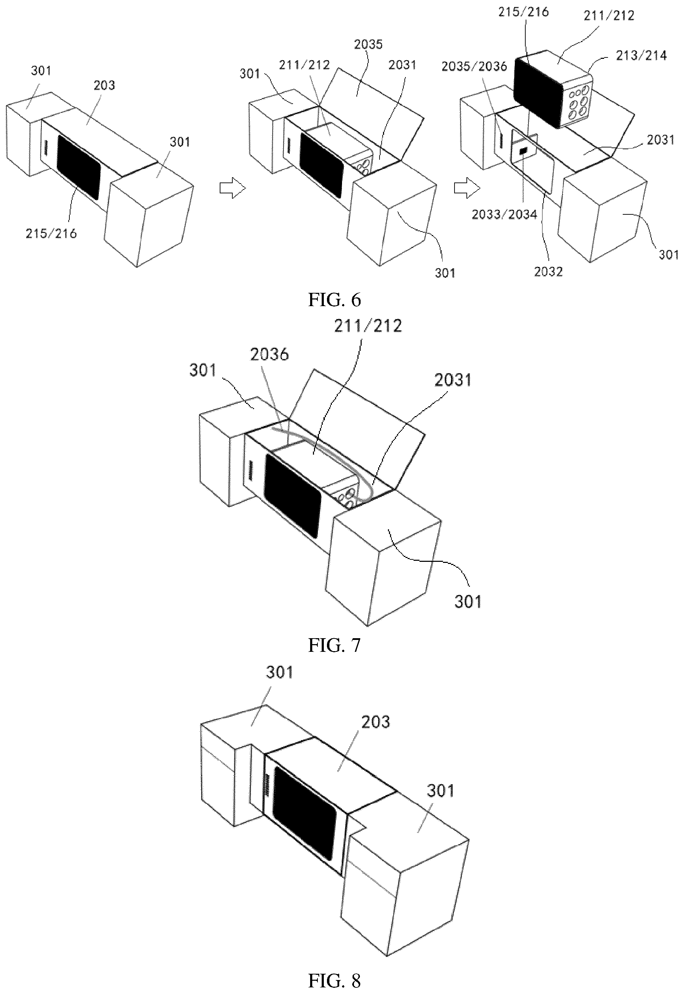

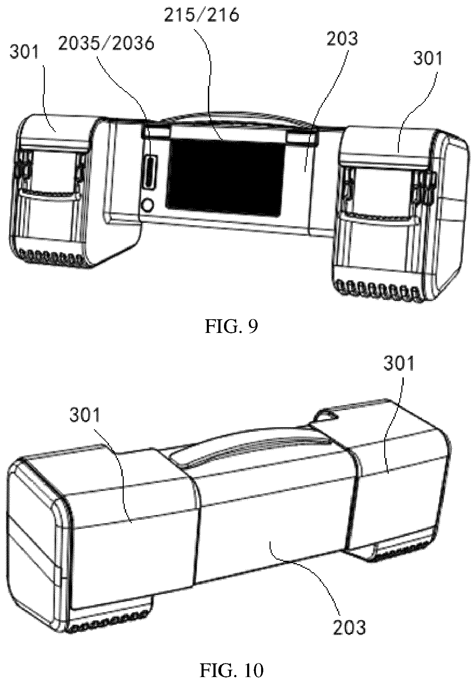

For this, please refer to FIGS. 6-13. In some embodiments, the monitoring apparatus 201 is also provided with a first storage member 301. The first storage member 301 is configured to store monitoring accessories (such as sensors and connecting cables), thereby avoiding disorderly placement of monitoring accessories that occupy already limited space, as well as affecting work of user. Moreover, the first storage member 301 is detachably connected with the monitoring apparatus 201, or the first storage member 301 is affixed to the monitoring apparatus 201, or the first storage member 301 and the monitoring apparatus 201 are integrally formed. The first storage member 301 can be transferred together with its stored monitoring accessories and the monitoring apparatus 201, making it convenient for user to carry.

The detachably connection, includes but is not limited to a clamping fixation, a magnetic fixation, a detachably adhering fixation, etc. The affixing, includes but is not limited to a suture fixation, a fixed adhering, welding (such as ultrasonic welding), etc. The first storage member 301 can be a box body made of fabric materials or other materials, or can be a plastic box.

The monitoring apparatus 201 at least includes a monitoring module 211, which is configured to implement monitoring functions. The monitoring module 211 includes a monitoring module housing 213, a control unit for receiving and processing monitoring data, a display unit 215 for displaying monitoring data, the aforementioned information transmission unit of the monitoring module, and other related components. That is, the monitoring module 211 can independently complete the functions of monitoring, detection, and monitoring information displaying. The control unit of the monitoring module and the information transmission unit of the monitoring module can be located inside the monitoring module housing 213, and the display unit 215 of the monitoring module can be exposed from the monitoring module housing 213 to display monitoring information. The control unit of the monitoring module is located inside the monitoring module housing 213, so it is not shown in the figure.

The monitoring apparatus 201 can mainly have a monitoring module 211, and at this time, the first storage member 301 can be detachably connected with the monitoring apparatus 201, or the first storage member 301 can be affixed to the monitoring apparatus 201, or the first storage member 301 and the monitoring apparatus 201 can be integrally formed. The first storage member 301 can be transferred together with its stored monitoring accessories and the monitoring apparatus 201, making it convenient for user to carry.

The monitoring module 211 is provided with a first docking structure which is detachably connected with the assembly structure. The first docking structure corresponds to the assembly structure 120 of the host 101, which includes but is not limited to a clamping structure, a magnetic structure, a structure that is capable of being detachably adhered, a screwing structure, a tight fitting structure, etc. Through the first docking structure, the monitoring module 211 can be directly assembled with the host 101, forming an integral structure with the host 101 for portability. In this embodiment, by directly connecting the monitoring module 211 to the host 101 in a detachably way, the volume of the monitoring apparatus 201 can be reduced, thereby reducing the space used by the host 101 to accommodate the monitoring apparatus 201, which is conducive to further reducing the volume of the host 101.

In an embodiment, the first storage member 301 can be detachably connected with the monitoring module 211, thereby achieving an integrated structure with the monitoring module 211, enabling the monitoring module 211 and the first storage member 301 to move together for convenient storage of the accessories of the monitoring module 211.

In other embodiments, please refer to FIGS. 6-13. The monitoring apparatus 201 also includes a second storage member 203, which is provided with a storage chamber 2031 for storing the monitoring module 211, and with a second docking structure which is detachably connected with the assembly structure 120. The storage chamber 2031 is configured to accommodate the monitoring module 211. In these embodiments, the monitoring module 211 is detachably connected with the host 101 through the second storage member 203. Wherein, the second docking structure corresponds to the assembly structure 120 of the host 101, and the second docking structure includes but is not limited to a clamping structure, a magnetic structure, a structure that is capable of being detachably adhered, a screwing structure, a tight fitting structure, etc.

Due to the use of the second storage member 203 for transfer, the monitoring module 211 only needs to be stored inside the second storage member 203. The monitoring module 211 may not have a separate first docking structure, thus reducing the manufacturing difficulty of the monitoring module 211. Even without changing the structure of the existing monitoring module 211, the existing monitoring module 211, which originally does not have an assembly function with the host 101, can be directly assembled in a way capable of being disassembled, with the host 101 through the second storage member 203, thus expanding the role of the existing monitoring module 211. The second storage member 203 can be a box body made of fabric materials, or other materials, or can be a plastic box. Typically, the second storage member 203 can have an openable cover 2035 to facilitate the assembly and disassembly of the monitoring module 211.

In order to facilitate the information display of the monitoring module 211, in some embodiments, when the monitoring module 211 is stored inside the storage chamber 2031, the display unit 215 of the monitoring module is not blocked by the second storage member 203. For example, please refer to FIGS. 6-8. In the illustrated embodiment, the second storage member 203 is provided with a window 2032 that can expose a display area of the display unit 215 of the monitoring module. The window 2032 can be a through opening or a transparent area made of transparent material. A surface area of this window 2032 is compatible with the display area of the display unit 215 of the monitoring module, preferably equal to or greater than a surface area of the display area, so as to fully display the display area and facilitate user to view information.

In addition to achieving transfer function in physical space, the second storage member 203 can also serve as a communication transfer structure between the monitoring module 211 and the host 101. For example, in some embodiments, the second storage member 203 is provided with an information transferring unit, which includes a docking part for the monitoring module 2033 and a host docking part 2035. The docking part for the monitoring module 2033 is in communicative connection with the information transmission unit of the monitoring module in the monitoring module 211, and the host docking part 2035 is in communicative connection with the host information transmission unit in the host 101.

Wherein, the information transferring unit can be a communication unit with a physical docking part 119 and/or a wireless communication unit that implements wireless communication. In some embodiments, both the docking part for the monitoring module 2033 and the host docking part 2035 can use metal contacts, plug-in interfaces, or other types of physical docking unit 119. The types of the docking part for the monitoring module 2033 and the host docking part 2035 may be different. For example, since the monitoring module 211 can be stored inside the second storage member 203 for use, the docking part for the monitoring module 2033 can be connected with the monitoring module 211 through cables to ensure the reliability of the connection. Of course, it can also be connected to the monitoring module 211 through metal contacts and plug interfaces. In order to implement rapid docking between the second storage member 203 and the host 101, metal contacts, plug-in interfaces, and other structures that can implement rapid docking can be selected. Of course, in other embodiments, the host docking part 2035 can also use cables to implement a more reliable connection with the host 101.

Of course, when the host 101 and the monitoring module 211 are connected through wireless communication, in some embodiments, the second storage member 203 may not have a communication transfer function. Of course, the wireless communicative connection between the host 101 and the monitoring module 211 does not conflict with the communication transfer function of the second storage member 203, and in some embodiments, both can also be used.

In addition, the information transferring unit can also be used as a transferring unit for power supply, such as the host 101 supplies power to the monitoring module 211 through the information transferring unit, or vice versa, the monitoring module 211 supplies power to the host 101 through the information transferring unit. When the information transferring unit is used as a power supply component, it can be implemented through wireless charging or wired connection. Of course, additional charging circuits can also be provided at the host 101, the second storage member 203, and the monitoring apparatus 201 to implement charging.

The second storage member 203 and the monitoring module 211 form an integral structure, and the user can carry the second storage member 203 and the monitoring module 211 together for movement. Please refer to FIG. 7. The second storage member 203 can not only store the monitoring module 211, but also some cable 2036 for the monitoring module 211. This cable 2036 can be either an accessory cable or other cable, such as power cable, signal cable, etc.

Of course, in an embodiment, the second storage member 203 includes multiple separated sub-storage members, which are integrally formed with, detachably connected with, or affixed to each other. These sub-storage members are divided into different spaces to classify and store different items. Of course, as shown in FIGS. 6 and 7, in some embodiments, the second storage member 203 may also have only one storage chamber 2031 for storing the monitoring module 211 and some cables thereof.

Please refer to FIGS. 6-13. In some embodiments, the first storage member 301 and the second storage member 203 are integrally formed with, detachably connected with, or affixed to each other. By using the second storage member 203, the first storage member 301 and the monitoring module 211 to form an integrated structure, the monitoring module 211 and the first storage member 301 can be moved together for convenient storage of the accessories of the monitoring module 211.

Furthermore, in some embodiments, the first storage member 301 can be arranged at at least one of: a rear side, a left side, and a right side of the monitoring apparatus 201 to reserve space at a front side of the monitoring apparatus 201, so that the monitoring apparatus 201 can dock with the host 101 from the front side. This docking method is more in line with functional habit of user. Moreover, the front-rear assembly of the host 101 and the monitoring apparatus 201 is also conducive to reducing the size of the entire device for defibrillation and monitoring in the left-right direction, avoiding occupying too much space in the width direction of the medical facility. Of course, in some embodiments, when the assembly of the monitoring apparatus 201 and the host 101 is not hindered, the first storage member 301 can also be arranged at the front side of the monitoring apparatus 201.

For example, in some embodiments, when the first storage member 301 is arranged at the monitoring module 211, the left and right sides of the monitoring module 211 are respectively provided with the first storage member 301, forming a left and right balanced structure. Please refer to FIGS. 6-13. In other embodiments, when the first storage member 301 is arranged at the second storage member 203, the left and right sides of the second storage member 203 are respectively provided with the first storage member 301, forming a left and right balanced structure. When the monitoring module 211 and the first storage member 301 are used separately from the host 101, these two structures are conducive to concentrating the center of gravity on the monitoring module 211 and the second storage member 203, making it more convenient to carry and use.

Please refer to FIGS. 6-13. In some embodiments, the first storage member 301 is arranged at both sides of the monitoring apparatus 201 and does not occupy the front and rear spaces the monitoring apparatus 201. With the front-rear coordination between the monitoring apparatus 201 and the host 101, the first storage member 301 can be designed to be larger or smaller as needed. The volume of the first storage member 301 is not affected by the volume of the host 101 and the monitoring apparatus 201, making it more flexible.

Of course, each first storage member 301 can also include multiple separated sub-storage members, which are integrally formed with, detachably connected with, or affixed to each other. Through these sub-storage members, various monitoring accessories can be classified and stored, making it more convenient to manage and retrieve the required monitoring accessories. In other embodiments, each first storage member 301 may also have only one storage chamber for unified storage of monitoring accessories.

Please refer to FIGS. 7 and 8. In some embodiments, on a cross-section which is along a front-rear direction and perpendicular to a bottom surface of the device for defibrillation and monitoring, a projection of the first storage member 301 at least partially overlaps with a projection of the host 101. At this time, the front side of the first storage member 301 protrudes from the second storage member 203 (or monitoring module 211) to form an concave structure between the second storage member 203 (or monitoring module 211) and the first storage member 301, which concave structure is used for inserting into the host 101. The concave structure fully utilizes the space on both sides of the host 101 and the monitoring apparatus 201 to arrange the first storage member 301, allowing the first storage member 301 to be designed larger to accommodate more monitoring accessories and ensuring that the first storage member 301 does not occupy the space for the host 101 and the monitoring apparatus 201, maintaining normal docking between the host 101 and the monitoring apparatus 201.

In order to implement the concave structure mentioned above, please refer to FIG. 7. In an embodiment, the second storage member 203 (or monitoring module 211) has a relatively large left-right width. The first storage member 301 has a square horizontal cross-section, and the first storage member 301 is fixed at both sides of the second storage member 203 (or monitoring module 211) to form the concave structure. Please refer to FIG. 8. In another embodiment, the second storage member 203 (which can also be the monitoring module 211) has a relatively small left-right width, the first storage member 301 has an L-shaped horizontal cross-section, and the first storage member 301 is fixed at both sides of the second storage member 203 (or monitoring module 211). This assembly form can arrange the first storage member 301 at both sides of the second storage member 203 (or monitoring module 211), so as to more fully utilize the space on both sides of the second storage member 203 (or monitoring module 211), such that the storage space of the first storage member 301 has been cleverly increased without changing the horizontal width of the integral module.

Of course, in other embodiments, on a cross-sectional plane, which is along a left-right direction and perpendicular to the bottom surface of the device for defibrillation and monitoring, a projection of the first storage member 301 does not overlap with a projection of the host 101 or partially overlaps with the projection of the host 101.

Usually, the monitoring apparatus 201 is also provided with an electrode sheet 410 and a cable for electrode sheet 420, which can be stored inside the first storage member 301 or the second storage member 203 mentioned above. Considering that the electrode sheet 410 is usually in a plate-like structure, so as to avoid causing the volume of the first storage member 301 or the second storage member 203 to be too bulky. In some embodiments, a third storage member 302 may also be included, which is configured to store the electrode sheet 410 and/or the cable for electrode sheet 420. Of course, the third storage member 302 can also be configured to store other accessories as needed, in addition to storing the electrode sheet 410 and/or cable for electrode sheet 420.

Wherein, the third storage member 302 is detachably connected with the monitoring apparatus 201. Optionally, the third storage member 302 is detachably connected with the host 101, or the third storage member 302 is affixed to the host 101, or the third storage member 302 and the host 101 are integrally formed.

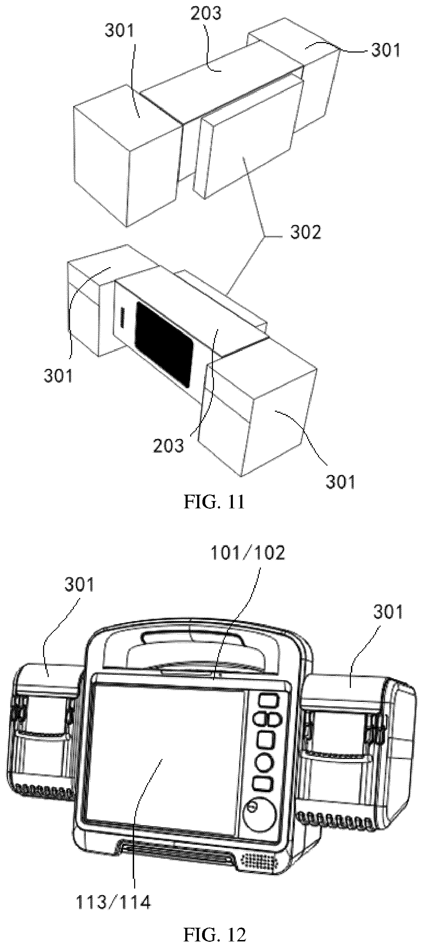

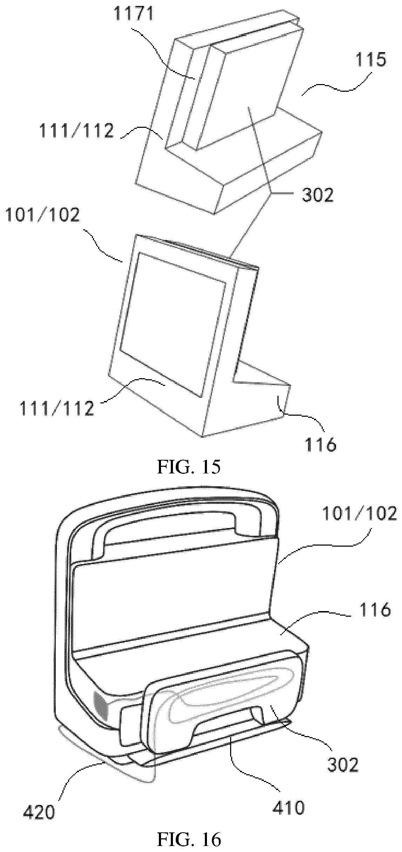

As shown in FIGS. 11-15, in some embodiments, when the third storage member 302 is configured to store the electrode sheet 410, the third storage member 302 may have a plate-like structure similar to the electrode sheet 410 to reduce thickness and avoid bulkiness. As shown in FIGS. 16 and 17, as well as FIGS. 19 and 20, in some embodiments, when the third storage member 302 is configured to store the cable for electrode sheet 420, the third storage member 302 may have a smaller design that is sufficient to store the cable for electrode sheet 420.

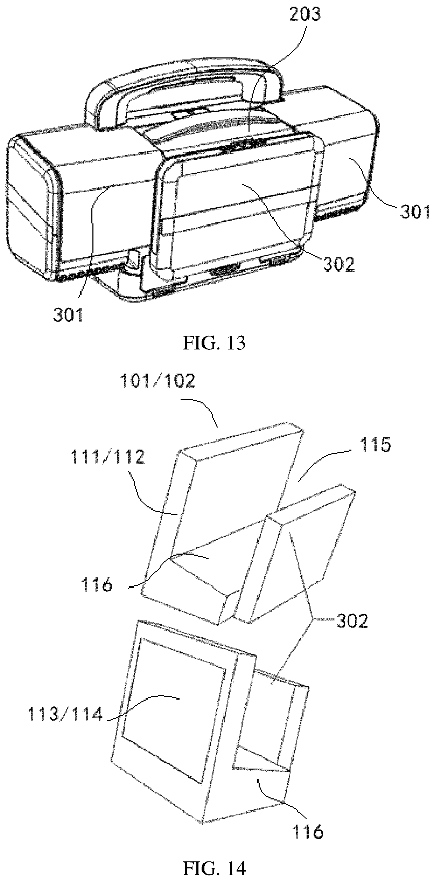

The third storage member 302 can be assembled with the host 101 or the monitoring apparatus 201, but due to the fact that the electrode sheet 410 is usually used in cooperation with the host 101, in some embodiments, the third storage member 302 is preferred to be arranged at the host 101. For example, please refer to FIGS. 14-17. In these embodiments, the third storage member 302 is arranged at the host 101.

In order to obtain a more compact structure, the third storage member 302 is arranged at the side surface 1171 located in front of the assembly area for extension module 115 and/or at the rear side of the support platform 116. Specifically, please refer to FIG. 14. In this embodiment, the third storage member 302 is arranged at the rear side of the support platform 116. The third storage member 302 can not only store the electrode sheet 410 and/or cable for electrode sheet 420, but also be configured to enclose the assembly area for extension module 115 at the rear side of the monitoring apparatus 201. Please refer to FIG. 15. In this embodiment, the third storage member 302 is arranged at the side surface 1171 in front of the assembly area for extension module 115, fully utilizing the space in the assembly area for extension module 115, without requiring to add the third storage member 302 in other positions. Please refer to FIGS. 16, 17, and 20. The third storage member 302 is also arranged at the rear side of the support platform 116 for storing the cable for electrode sheet 420. At this point, if the storage of the electrode sheet 410 is not considered, the height of the third storage member 302 can be designed to be lower. In these embodiments, the third storage member 302 is arranged in parallel with the host display unit 113.

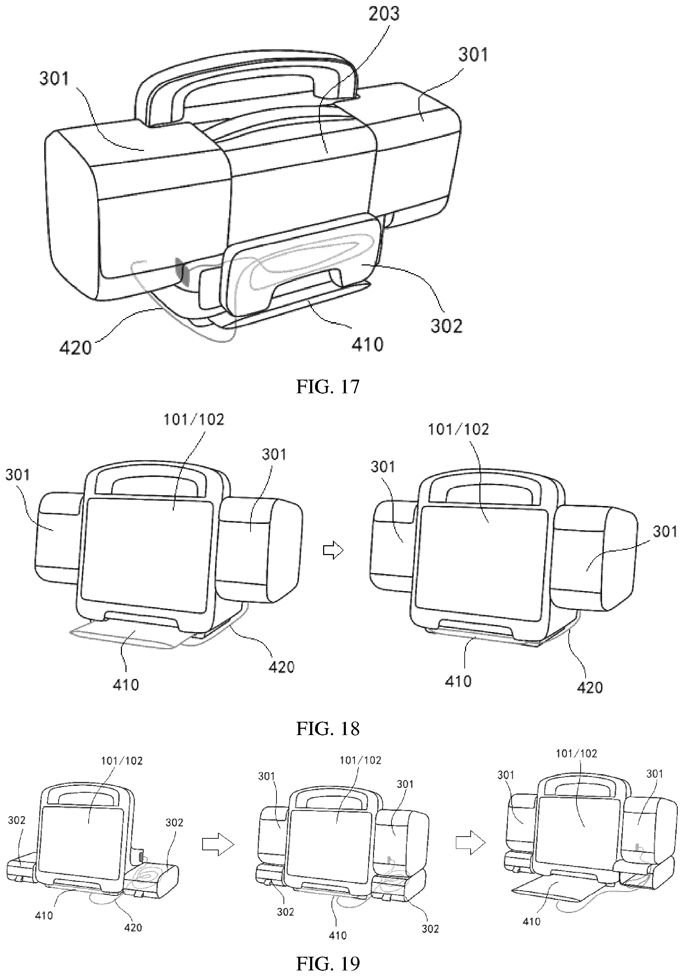

In addition, the third storage member 302 can also be arranged at other positions of the host 101, such as at both sides of the host 101. Please refer to FIG. 19. In an embodiment, the third storage member 302 is arranged at the left and right sides (or one side) of the host 101.

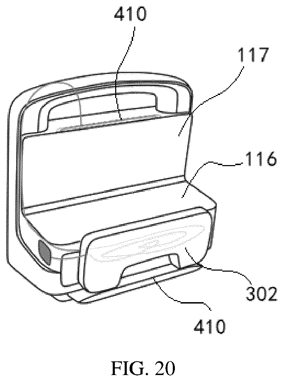

When the third storage member 302 is configured to store the cable for electrode sheet 420, a storage chamber for electrode sheet can also be arranged at the host 101 to store the electrode sheet 410. For example, please refer to FIGS. 18 and 19. In these embodiments, the storage chamber for electrode sheet can be arranged at the bottom of the host 101, with the storage chamber for electrode sheet opening towards the front side for the electrode sheet 410 to be inserted into and taken out. For example, please refer to FIG. 20. In this embodiment, the side part 117 of the host 101 facing the assembly area for extension module 115 can be provided with a storage chamber for electrode sheet, which opens upwards for the electrode sheet 410 to be inserted into and taken out. Please refer to FIGS. 16, 17, and 20. In some embodiments, the storage chamber for electrode sheet can also be arranged at a rear part of the host 101, with the storage chamber for electrode sheet opening towards the front side for the electrode sheet 410 to be inserted into and taken out. In addition, the storage chamber for electrode sheet can also be arranged at other positions of the host 101.

When the third storage member 302 that stores the cable for electrode sheet 420 is provided, the cable for electrode sheet 420 is also pre-connected with the host 101, so it is not necessary to connect it on-site during emergency treatment.

In addition, the third storage member 302 can also be assembled with the monitoring apparatus 201. Please refer to FIGS. 11-13. In these embodiments, the third storage member 302 is located behind the second storage member 203 (or the monitoring module 211). The third storage member 302 is detachably connected with the second storage member 203 (or monitoring module 211), for easy disassembly and use at any time.

Of course, the third storage member 302 can also be arranged at other positions of the second storage member 203 (or monitoring module 211) or at the first storage member 301.

Furthermore, this disclosure further provides a monitoring component, which includes a monitoring module 211 and a second storage member 203. The monitoring module 211 and the second storage member 203 can adopt the structure shown in the above embodiments. The second storage member 203 is configured to store the monitoring module 211, that is, the monitoring module 211 can be placed inside the second storage member 203. Moreover, the second storage member 203 is further provided with a second docking structure, through which the second storage member 203 can be assembled, together with the monitoring module 211, to other apparatus, such as the host 101 or other apparatus mentioned above, so that the monitoring module 211 can form an assembly structure with other apparatus, which assembly structure is capable of being disassembled.

Furthermore, this disclosure further provides another monitoring component, which includes a monitoring module 211 and a first storage member 301. The monitoring module 211 and the first storage member 301 can adopt the structure shown in the above embodiments. The first storage member 301 is detachably connected with the monitoring module 211, or the first storage member 301 is affixed to the monitoring module 211, or the first storage member 301 and the monitoring module 211 are integrally formed. The first storage member 301 is configured to store monitoring accessories (such as sensors and connecting cables), thereby avoiding disorderly placement of monitoring accessories that occupy already limited space, as well as affecting work of user.

On the other hand, some embodiments of this disclosure further provide a device for defibrillation and monitoring, which differs from the devices for defibrillation and monitoring shown in the above embodiments in that the device for defibrillation and monitoring includes a display apparatus 102 and an apparatus for defibrillation and monitoring 202. The display apparatus 102 is mainly configured to display information. The apparatus for defibrillation and monitoring 202 includes an integrated machine for defibrillation and monitoring 212, which can independently complete monitoring and defibrillation operations. Of course, the display apparatus 102 can be used for display purposes, so as to display defibrillation information and monitoring information. However, in some embodiments, the display apparatus 102 can also be configured to control the integrated machine for defibrillation and monitoring 212 to implement monitoring and/or defibrillation operations.

Please refer to FIGS. 1-20. In each embodiment shown, the display apparatus 102 may have the same or similar shape and structure as the host 101, so the two are labeled together. Other reference numbers labeled together in the following description are interpreted accordingly. The apparatus for defibrillation and monitoring 202 can have the same or similar shape and structure as the monitoring apparatus 201, and the integrated machine for defibrillation and monitoring 212 can have the same or similar shape and structure as the monitoring module 211.

The display apparatus 102 includes a display apparatus housing 112 (in the embodiments shown in FIGS. 1-20, the display apparatus housing 112 may have the same or similar shape and structure as the host housing 111), a display unit 114 (in the embodiments shown in FIGS. 1-20, the display unit 114 of the display apparatus may have the same or similar shape and structure as the host display unit 113) and an information transmission unit for information transmission (the information transmission unit of the display apparatus may have the same or similar shape and structure as the host information transmission unit).

In an embodiment, the integrated machine for defibrillation and monitoring 212 includes an integrated machine housing 214 (in the embodiments shown in FIGS. 1-20, the integrated machine housing 214 may have the same or similar shape and structure as the monitoring module housing 213), an display unit 216 for displaying information (in the embodiments shown in FIGS. 1-20, the display unit 216 of the integrated machine may have the same or similar shape and structure as the host display unit 113), a high-voltage capacitor, and a detection circuit for thoracic bioimpedance, a charging circuit, a discharging circuit, an information transmission unit for information transmission, a monitoring parameter circuit, and a control unit.

Wherein, the integrated machine housing 214 is provided with a second accommodating chamber, wherein the high-voltage capacitor, the charging circuit, the discharging circuit, the information transmission unit of the integrated machine, the control unit of the integrated machine, and the monitoring parameter circuit are all located inside the second accommodating chamber, so they are not shown in the attached drawings. The display unit 114 of the display apparatus is arranged at the front side of the display apparatus housing 112, and is configured to display the defibrillation information and monitoring information transmitted by the apparatus for defibrillation and monitoring 202.

In an embodiment, the integrated machine for defibrillation and monitoring 212 can include a detection circuit for thoracic bioimpedance, which is configured to acquire detection data of thoracic bioimpedance. The control unit of the integrated machine receives detection data of thoracic bioimpedance and controls the charging and discharging circuits to complete the charging and discharging process of the high-voltage capacitor.

The information transmission unit of the integrated machine can have the same or similar structure as the information transmission unit of the monitoring module shown in the above embodiments. The information transmission unit of the integrated machine is in communicative connection with the information transmission unit of the display apparatus to implement unidirectional information transmission or bidirectional information transmission. The control unit of the integrated machine is also configured to control the display unit 216 of the integrated machine to display defibrillation information and monitoring information.

The apparatus for defibrillation and monitoring 202 can implement monitoring function regardless of whether the apparatus for defibrillation and monitoring 202 is assembled with the assembly structure 120 or not. The integrated machine for defibrillation and monitoring 212 can independently complete monitoring and defibrillation operations. The display apparatus housing 112 is provided with the assembly structure 120. When a larger display window is needed for displaying, the display apparatus 102 and the apparatus for defibrillation and monitoring 202 can be combined into a whole and used together to display defibrillation information and monitoring information through the display apparatus 102. But when it is necessary to reduce the weight and volume of the device, only the apparatus for defibrillation and monitoring 202 can be carried to the medical assistance facilities. Therefore, users can flexibly choose the devices they carry according to their requirements, improving the convenience of use.

The display apparatus 102 and the assembly structure 120 can adopt the assembly structure 120 and the display apparatus 102 mentioned in any above embodiments. The apparatus for defibrillation and monitoring 202 is assembled with the assembly structure 120 for realizing the assembly between the display apparatus 102 and the apparatus for defibrillation and monitoring 202, which assembly is capable of being disassembled. The connection manner and/or assembly manner between the display apparatus 102 and the apparatus for defibrillation and monitoring 202, can adapt any connection manner and/or assembly manner capable of being disconnected and/or disassembled, between the host 101 and the monitoring apparatus 201 mentioned in any above embodiments.

For example, the apparatus for defibrillation and monitoring 202 is also provided with a first docking structure as shown in the above embodiments for docking with the assembly structure 120 of the display apparatus 102.

For example, the display apparatus 102 may have an assembly area for extension module 115 as shown in the above embodiments. When the apparatus for defibrillation and monitoring 202 is assembled with the assembly structure 120, at least a portion of the apparatus for defibrillation and monitoring 202 is stored within the assembly area for extension module 115.

Furthermore, please refer to FIGS. 6-20. In some embodiments, the apparatus for defibrillation and monitoring 202 is further provided with a first storage member 301, which is configured to store defibrillation and/or monitoring accessories, and is detachably connected with the apparatus for defibrillation and monitoring 202, or is affixed to the apparatus for defibrillation and monitoring 202, or is integrally formed with the apparatus for defibrillation and monitoring 202. The first storage member 301 is configured to store defibrillation and/or monitoring accessories (such as monitoring sensors and connecting cables, defibrillation electrode sheet 410, electrode plate, etc.), so as to avoid disorderly placement of accessories that occupy already limited space and avoid affecting work of user. The first storage member 301 can be the first storage member 301 shown in the above embodiments.

Furthermore, please refer to FIGS. 6-20. In some embodiments, the apparatus for defibrillation and monitoring 202 further includes a second storage member 203, which is provided with a storage space for storing the integrated machine for defibrillation and monitoring 212, and with a second docking structure which is detachably connected with the assembly structure 120. The second storage member 203 can be the second storage member 203 shown in the above embodiments. The second storage member 203 can also be provided with an information transferring unit, which includes a docking part 2034 for the integrated machine and a docking part 2036 for the display apparatus. The docking part 2034 for the integrated machine is in communicative connection with the information transmission unit of the integrated machine in the integrated machine for defibrillation and monitoring 212, and the docking part 2036 for the display apparatus is in communicative connection with the information transmission unit of the display apparatus.

Furthermore, please refer to FIGS. 11-20. In some embodiments, a third storage member 302 is further provided, which is configured to store the electrode sheet 410 and/or the cable for electrode sheet 420. The third storage member 302 can be detachably connected with the apparatus for defibrillation and monitoring 202 or the display apparatus 102, or the third storage member 302 can be affixed to the apparatus for defibrillation and monitoring 202 or the display apparatus 102, or the third storage member 302 can be integrally formed with the apparatus for defibrillation and monitoring 202 or the display apparatus 102. The third storage member 302 can be the third storage member 302 shown in the above embodiments.

Furthermore, this disclosure further provides a component for defibrillation and monitoring, which includes an integrated machine for defibrillation and monitoring 212 and a second storage member 203. The second storage member 203 is configured to store the integrated machine for defibrillation and monitoring 212, that is, the integrated machine for defibrillation and monitoring 212 can be placed into the second storage member 203. Moreover, the second storage member 203 is further provided with a second docking structure, through which the second storage member 203 can be assembled, together with the integrated machine for defibrillation and monitoring 212, to other apparatus, such as the display apparatus 102 mentioned above, so that the integrated machine for defibrillation and monitoring 212 can form an assembly structure with other apparatus, which assembly structure is capable of being disassembled.

Furthermore, this disclosure further provides another component for defibrillation and monitoring, which includes an integrated machine for defibrillation and monitoring 212 and a first storage member 301. The first storage member 301 is detachably connected with the integrated machine for defibrillation and monitoring 212, or the first storage member 301 is affixed to the integrated machine for defibrillation and monitoring 212, or the first storage member 301 and the integrated machine for defibrillation and monitoring 212 are integrally formed. The first storage member 301 is configured to store defibrillation and/or monitoring accessories (such as monitoring sensors and connecting cables, defibrillation electrode sheet 410, electrode plate, etc.), so as to avoid disorderly placement of accessories that occupy already limited space and avoid affecting work of user.

The above applies specific embodiments to illustrate this disclosure, only for the purpose of assisting in understanding it and not to limit it. For those skilled in the technical field to which this disclosure belongs, based on the ideas of this disclosure, several simple deductions, deformations, or substitutions can also be made.

## Claims

1. A device for defibrillation and monitoring, comprising: a host (101), which comprises a host housing (111), a host control unit, a host display unit (113) which is configured to display defibrillation information, a high-voltage capacitor, a charging circuit, a discharging circuit, and a host information transmission unit which is configured to transmit information; wherein the host housing (111) is provided with an assembly structure (120); wherein the host control unit is configured to control the charging circuit and the discharging circuit to complete a charging and discharging process of the high-voltage capacitor, and configured to generate the defibrillation information; wherein the host housing (111) is also provided with a first accommodating chamber; wherein the host control unit, the high-voltage capacitor, the charging circuit and the discharging circuit are all located inside the first accommodating chamber, and the host display unit (113) is arranged at a front side of the host housing (111);
a monitoring apparatus (201), which is assembled with the assembly structure (120) and capable of being disassembled from the assembly structure (120); wherein the monitoring apparatus (201) is capable of implementing a monitoring function regardless of whether it is assembled with the assembly structure (120) or not, wherein the host (101) is in communicative connection with the monitoring apparatus (201) through the host information transmission unit to implement unidirectional information transmission or bidirectional information transmission;
a first storage member (301), which is configured to store a monitoring accessory, **characterised in that** the first storage member (301) is detachably connected with the monitoring apparatus (201), or the first storage member (301) is affixed to the monitoring apparatus (201), or the first storage member (301) and the monitoring apparatus (201) are integrally formed; wherein the first storage member (301) is capable of being transferred together with the monitoring accessory and the monitoring apparatus (201).

2. The device for defibrillation and monitoring according to claim 1, **characterised in that**, at least two surfaces of the host housing define an assembly area for extension module; wherein at least a portion of the monitoring apparatus is stored inside the assembly area for extension module, when the monitoring apparatus is assembled with the assembly structure.

3. The device for defibrillation and monitoring according to claim 1 or 2, **characterised in that**, the monitoring apparatus is arranged at at least one of: a left side, a right side, a rear side, an upper side, and a lower side of the host display unit, when the monitoring apparatus is assembled with the assembly structure;
preferably, the monitoring apparatus is arranged at the rear side of the host display unit, when the monitoring apparatus is assembled with the assembly structure; or the monitoring apparatus is capable of being placed into and/or taken out of an assembly area for extension module, from at least one of: a rear side, a left side, a right side, an upper side, and a lower side of the assembly area for extension module; or at least one of: a rear side, a left side, a right side, an upper side, and a lower side of an assembly area for extension module, is open, such that the monitoring apparatus is capable of being placed into and/or taken out of the assembly area for extension module, from the at least one open side; wherein the at least one open side is exposed or provided with a barrier structure which is capable of being opened and closed.

4. The device for defibrillation and monitoring according to claim 2, **characterised in that**, the host housing is provided with a support platform, which comprises a support surface, wherein the at least two surfaces of the host housing, which define the assembly area for extension module, comprise the support surface; wherein the assembly area for extension module is arranged at an upper side of the support platform, and the support surface is configured to support the monitoring apparatus;
preferably, the support platform is arranged at a rear side of the host display unit, and the monitoring apparatus is capable of being placed into and/or taken out of the support platform, from at least one of: a rear side, a left side, a right side and an upper side of the assembly area for extension module; or a first angle is formed between the host display unit and a vertical direction, and the support surface is inclined downward in a front-rear direction for the first angle, when the device for defibrillation and monitoring is placed horizontally.

5. The device for defibrillation and monitoring according to claim 2, **characterised in that**, the at least two surfaces of the host housing, which define the assembly area for extension module, comprise a side surface which is located in front of the assembly area for extension module, wherein the host information transmission unit comprises a docking part, which is connected with the monitoring apparatus, wherein the docking part is arranged at the side surface which is located in front of the assembly area for extension module.

6. The device for defibrillation and monitoring according to any one of claims 1-5, **characterised in that**, the host further comprises a detection circuit for thoracic bioimpedance, which is configured to acquire detection data of thoracic bioimpedance; wherein the host control unit is configured to receive the detection data of thoracic bioimpedance, so as to control the charging circuit and the discharging circuit to complete the charging and discharging process of the high-voltage capacitor; or
the first storage member comprises multiple separated sub-storage members, which are integrally formed with, detachably connected with, or affixed to each other.

7. The device for defibrillation and monitoring according to any one of claims 1-6, **characterised in that**, the monitoring apparatus comprises a monitoring module; wherein the monitoring module comprises a control unit, which is configured to receive and process monitoring data, a display unit, which is configured to display the monitoring data, and an information transmission unit;
wherein the information transmission unit of the monitoring module is in communicative connection with the host information transmission unit to implement unidirectional information transmission or bidirectional information transmission.

8. The device for defibrillation and monitoring according to claim 7, **characterised in that**, the monitoring module is provided with a first docking structure which is detachably connected with the assembly structure;
preferably, the first storage member is detachably connected with the monitoring module.

9. The device for defibrillation and monitoring according to claim 7, **characterised in that**, the monitoring apparatus further comprises a second storage member; wherein the second storage member is provided with a storage chamber, which is configured to store the monitoring module, and with a second docking structure which is detachably connected with the assembly structure.

10. The device for defibrillation and monitoring according to claim 9, **characterised in that**,
the display unit of the monitoring module is not blocked by the second storage member, when the monitoring module is stored inside the storage chamber; wherein the second storage member is provided with an information transferring unit, wherein the information transferring unit comprises a docking part for the monitoring module and a host docking part, wherein the docking part for the monitoring module is in communicative connection with the information transmission unit of the monitoring module, and the host docking part is in communicative connection with the host information transmission unit; or
the second storage member comprises multiple separated sub-storage members, which are integrally formed with, detachably connected with, or affixed to each other; or
the first storage member and the second storage member are integrally formed, or the first storage member is detachably connected with the second storage member, or the first storage member is affixed to the second storage member.

11. The device for defibrillation and monitoring according to any one of claims 1-10, **characterised in that**, the first storage member is arranged at at least one of: a rear side, a left side, and a right side of the monitoring apparatus;
preferably, both left and right sides of a monitoring module or of a second storage member, are provided with the first storage member.

12. The device for defibrillation and monitoring according to claim 1, **characterised in that**, a projection of the first storage member on a cross-sectional plane at least partially overlaps with a projection of the host on said cross-sectional plane, wherein said cross-sectional plane is along a front-rear direction and perpendicular to a bottom surface of the device for defibrillation and monitoring; or
a projection of the first storage member on a cross-sectional plane, partially overlaps or does not overlap with a projection of the host on said cross-sectional plane, wherein said cross-sectional plane is along a left-right direction and perpendicular to a bottom surface of the device for defibrillation and monitoring.

13. The device for defibrillation and monitoring according to any one of claims 1, 4 or 5, **characterised in that**, further comprising a third storage member, which is configured to store electrode sheet(s) and/or cable(s) for electrode sheet(s);
wherein, the third storage member is detachably connected with the monitoring apparatus; or the third storage member is detachably connected with the host, or the third storage member is affixed to the host, or the third storage member and the host are integrally formed;
preferably, the third storage member is arranged at a side surface which is located in front of an assembly area for extension module and/or at a rear side of a support platform; or the third storage member is parallel with the host display unit.

## Patentansprüche

1. Eine Vorrichtung zur Defibrillation und Überwachung, die Folgendes umfasst: einen Host (101), der ein Hauptgehäuse (111), eine Host-Steuereinheit, eine Host-Anzeigeeinheit (113), die zur Anzeige von Defibrillationsinformationen ausgelegt ist, einen Hochspannungskondensator, eine Ladeschaltung, eine Entladeschaltung sowie eine Host-Informationsübertragungseinheit, die zur Übertragung von Informationen ausgelegt ist; wobei das Hauptgehäuse (111) mit einer Baugruppenstruktur (120) versehen ist; wobei die Host-Steuereinheit dazu ausgelegt ist, die Ladeschaltung und die Entladeschaltung zu steuern, um einen Lade- und Entladevorgang des Hochspannungskondensators durchzuführen, und weiterhin dazu ausgelegt ist, die Defibrillationsinformationen zu erzeugen; wobei das Hauptgehäuse (111) zudem mit einer ersten Aufnahmekammer versehen ist; wobei sich die Host-Steuereinheit, der Hochspannungskondensator, die Ladeschaltung und die Entladeschaltung alle innerhalb der ersten Aufnahmekammer befinden und die Host-Anzeigeeinheit (113) an einer Vorderseite des Hauptgehäuses (111) angeordnet ist;
ein Überwachungsgerät (201), das an der Baugruppenstruktur (120) montiert ist und von dieser wieder demontiert werden kann; wobei das Überwachungsgerät (201) in der Lage ist, eine Überwachungsfunktion auszuführen, unabhängig davon, ob es an der Baugruppenstruktur (120) montiert ist oder nicht, wobei der Host (101) über die Host-Informationsübertragungseinheit in Kommunikationsverbindung mit dem Überwachungsgerät (201) steht, um eine unidirektionale oder bidirektionale Informationsübertragung durchzuführen;
ein erstes Aufnahmeelement (301), das zum Aufnehmen eines Überwachungszubehörs ausgelegt ist, **dadurch gekennzeichnet, dass** das erste Aufnahmeelement (301) lösbar mit dem Überwachungsgerät (201) verbunden ist oder das erste Aufnahmeelement (301) am Überwachungsgerät (201) befestigt ist, oder das erste Aufnahmeelement (301) und das Überwachungsgerät (201) einstückig ausgebildet sind; wobei das erste Aufnahmeelement (301) zusammen mit dem Überwachungszubehör und dem Überwachungsgerät (201) transportiert werden kann.

2. Die Vorrichtung zur Defibrillation und Überwachung nach Anspruch 1, **dadurch gekennzeichnet, dass** mindestens zwei Flächen des Hauptgehäuses einen Montagebereich für ein Erweiterungsmodul definieren; wobei mindestens ein Teil des Überwachungsgeräts innerhalb des Montagebereichs für das Erweiterungsmodul untergebracht ist, wenn das Überwachungsgerät an der Montagestruktur montiert ist.

3. Die Vorrichtung zur Defibrillation und Überwachung nach Anspruch 1 oder 2, **dadurch gekennzeichnet, dass** das Überwachungsgerät an mindestens einer der folgenden Seiten der Host-Anzeigeeinheit angeordnet ist: der linken Seite, der rechten Seite, der Rückseite, der Oberseite und der Unterseite, wenn das Überwachungsgerät in die Baugruppe eingebaut ist;
vorzugsweise das Überwachungsgerät an der Rückseite der Host-Anzeigeeinheit angeordnet ist, wenn das Überwachungsgerät in die Baugruppenstruktur eingebaut ist; oder das Überwachungsgerät in einen Einbaubereich für Erweiterungsmodule eingesetzt und/oder aus diesem entnommen werden kann, und zwar von mindestens einer der folgenden Seiten: einer Rückseite, einer linken Seite, einer rechten Seite, einer Oberseite und einer Unterseite des Einbaubereichs für Erweiterungsmodule; oder mindestens eine der folgenden Seiten - Rückseite, linke Seite, rechte Seite, Oberseite und Unterseite - eines Einbaubereichs für Erweiterungsmodule offen ist, sodass das Überwachungsgerät über die mindestens eine offene Seite in den Einbaubereich für Erweiterungsmodule eingesetzt und/oder aus diesem entnommen werden kann; wobei die mindestens eine offene Seite freiliegt oder mit einer Barrierevorrichtung versehen ist, die geöffnet und geschlossen werden kann.

4. Die Vorrichtung zur Defibrillation und Überwachung nach Anspruch 2, **dadurch gekennzeichnet, dass** das Hauptgehäuse mit einer Auflageplattform versehen ist, die eine Auflagefläche umfasst, wobei die mindestens zwei Flächen des Hauptgehäuses, die den Montagebereich für das Erweiterungsmodul begrenzen, die Auflagefläche bilden; wobei der Montagebereich für das Erweiterungsmodul an einer Oberseite der Trägerplattform angeordnet ist und die Auflagefläche so ausgebildet ist, dass sie das Überwachungsgerät trägt;
vorzugsweise die Auflageplattform an einer Rückseite der Hauptanzeigeeinheit angeordnet ist, und das Überwachungsgerät von mindestens einer der folgenden Seiten - einer Rückseite, einer linken Seite, einer rechten Seite und einer Oberseite des Montagebereichs für das Erweiterungsmodul - in die Auflageplattform eingesetzt und/oder aus dieser entnommen werden kann; oder zwischen der Hauptanzeigeeinheit und einer vertikalen Richtung ein erster Winkel gebildet wird und die Auflagefläche in einer Front-Heck-Richtung um den ersten Winkel nach unten geneigt ist, wenn die Vorrichtung zur Defibrillation und Überwachung horizontal angeordnet ist.

5. Die Vorrichtung zur Defibrillation und Überwachung nach Anspruch 2, **dadurch gekennzeichnet, dass** die mindestens zwei Flächen des Hauptgehäuses, die den Montagebereich für das Erweiterungsmodul begrenzen, eine Seitenfläche umfassen, die sich vor dem Montagebereich für das Erweiterungsmodul befindet, wobei die Haupt-Informationsübertragungseinheit ein Andockteil umfasst, das mit dem Überwachungsgerät verbunden ist, wobei das Andockteil an der Seitenfläche angeordnet ist, die sich vor dem Montagebereich für das Erweiterungsmodul befindet.

6. Die Vorrichtung zur Defibrillation und Überwachung nach einem der Ansprüche 1 bis 5, **dadurch gekennzeichnet, dass** der Host ferner eine Erfassungsschaltung für die thorakale Bioimpedanz umfasst, die so ausgelegt ist, dass sie Erfassungsdaten der thorakalen Bioimpedanz erfasst; wobei die Host-Steuereinheit so ausgelegt ist, dass sie die Erfassungsdaten der thorakalen Bioimpedanz empfängt, um die Ladeschaltung und die Entladeschaltung so zu steuern, dass der Lade- und Entladevorgang des Hochspannungskondensators abgeschlossen wird; oder
das erste Aufnahmeelement mehrere voneinander getrennte Teilaufnahmeelemente umfasst, die einstückig ausgebildet, lösbar miteinander verbunden oder aneinander befestigt sind.

7. Die Vorrichtung zur Defibrillation und Überwachung nach einem der Ansprüche 1 bis 6, **dadurch gekennzeichnet, dass** das Überwachungsgerät ein Überwachungsmodul umfasst;
wobei das Überwachungsmodul eine Steuereinheit, die zum Empfangen und Verarbeiten von Überwachungsdaten ausgelegt ist, eine Anzeigeeinheit, die zum Anzeigen der Überwachungsdaten ausgelegt ist, und eine Informationsübertragungseinheit umfasst;
wobei die Informationsübertragungseinheit des Überwachungsmoduls in Kommunikationsverbindung mit der Host-Informationsübertragungseinheit steht, um eine unidirektionale oder bidirektionale Informationsübertragung durchzuführen.

8. Die Vorrichtung zur Defibrillation und Überwachung nach Anspruch 7, **dadurch gekennzeichnet, dass** das Überwachungsmodul mit einer ersten Andockstruktur versehen ist, die lösbar mit der Baugruppenstruktur verbunden ist;
das erste Aufnahmeelement vorzugsweise lösbar mit dem Überwachungsmodul verbunden ist.

9. Die Vorrichtung zur Defibrillation und Überwachung nach Anspruch 7, **dadurch gekennzeichnet, dass** das Überwachungsgerät ferner ein zweites Aufnahmeelement umfasst; wobei das zweite Aufnahmeelement mit einer Aufnahmekammer, die zur Aufnahme des Überwachungsmoduls ausgelegt ist, sowie mit einer zweiten Andockstruktur versehen ist, die lösbar mit der Baugruppenstruktur verbunden ist.

10. Die Vorrichtung zur Defibrillation und Überwachung nach Anspruch 9, **dadurch gekennzeichnet, dass**
die Anzeigeeinheit des Überwachungsmoduls nicht durch das zweite Aufnahmeelement verdeckt wird, wenn das Überwachungsmodul in der Aufnahmekammer untergebracht ist; wobei das zweite Aufnahmeelement mit einer Informationsübertragungseinheit versehen ist, wobei die Informationsübertragungseinheit ein Andockteil für das Überwachungsmodul und ein Host-Andockteil umfasst, wobei das Andockteil für das Überwachungsmodul in Kommunikationsverbindung mit der Informationsübertragungseinheit des Überwachungsmoduls steht und das Host-Andockteil in Kommunikationsverbindung mit der Host-Informationsübertragungseinheit steht; oder
das zweite Aufnahmeelement mehrere voneinander getrennte Teilaufnahmeelemente umfasst, die einstückig ausgebildet, lösbar miteinander verbunden oder aneinander befestigt sind; oder
das erste Aufnahmeelement und das zweite Aufnahmeelement einstückig ausgebildet sind, oder das erste Aufnahmeelement lösbar mit dem zweiten Aufnahmeelement verbunden ist, oder das erste Aufnahmeelement am zweiten Aufnahmeelement befestigt ist.

11. Die Vorrichtung zur Defibrillation und Überwachung nach einem der Ansprüche 1 bis 10, **dadurch gekennzeichnet, dass** das erste Aufnahmeelement an mindestens einer der folgenden Stellen angeordnet ist: einer Rückseite, einer linken Seite und einer rechten Seite des Überwachungsgeräts;
vorzugsweise sowohl die linke als auch die rechte Seite eines Überwachungsmoduls oder eines zweiten Aufnahmeelements mit dem ersten Aufnahmeelement versehen sind.

12. Die Vorrichtung zur Defibrillation und Überwachung nach Anspruch 1, **dadurch gekennzeichnet, dass** sich ein Vorsprung des ersten Aufnahmeelements auf einer Querschnittsebene zumindest teilweise mit einem Vorsprung des Trägerelements auf dieser Querschnittsebene überlappt, wobei die Querschnittsebene in einer Vor-Rück-Richtung verläuft und senkrecht zu einer Bodenfläche der Vorrichtung zur Defibrillation und Überwachung steht; oder
sich ein Vorsprung des ersten Aufnahmeelements auf einer Querschnittsebene teilweise mit einem Vorsprung des Gehäuses auf dieser Querschnittsebene überlappt oder nicht überlappt, wobei die Querschnittsebene in einer Links-Rechts-Richtung und senkrecht zu einer Unterseite des Geräts zur Defibrillation und Überwachung verläuft.

13. Die Vorrichtung zur Defibrillation und Überwachung nach einem der Ansprüche 1, 4 oder 5, **dadurch gekennzeichnet, dass** sie ferner ein drittes Aufnahmeelement umfasst, das zur Aufbewahrung von Elektrodenfolien und/oder Kabeln für Elektrodenfolien ausgebildet ist;
wobei das dritte Aufnahmeelement lösbar mit dem Überwachungsgerät verbunden ist; oder das dritte Aufnahmeelement lösbar mit dem Host verbunden ist, oder das dritte Aufnahmeelement am Host befestigt ist, oder das dritte Aufnahmeelement und der Host einstückig ausgebildet sind;
vorzugsweise das dritte Aufnahmeelement an einer Seitenfläche angeordnet ist, die sich vor einem Einbaubereich für ein Erweiterungsmodul und/oder an einer Rückseite einer Trägerplattform befindet; oder das dritte Aufnahmeelement parallel zur Anzeigeeinheit des Hosts verläuft.

## Revendications

1. Dispositif de défibrillation et de surveillance, comprenant : un hôte (101), lequel comprend un logement hôte (111), une unité de commande hôte, une unité d'affichage hôte (113) laquelle est configurée pour afficher des informations de défibrillation, un condensateur haute tension, un circuit de charge, un circuit de décharge, et une unité de transmission d'informations hôte laquelle est configurée pour transmettre des informations ; où le logement hôte (111) est doté d'une structure d'assemblage (120) ; où l'unité de commande hôte est configurée pour commander au circuit de charge et au circuit de décharge de réaliser un processus de charge et de décharge du condensateur haute tension, et configurée pour générer les informations de défibrillation ; où le logement hôte (111) est également doté d'une première chambre de réception ; où l'unité de commande hôte, le condensateur haute tension, le circuit de charge et le circuit de décharge sont tous situés dans la première chambre de réception, et l'unité d'affichage hôte (113) est agencée au niveau d'un côté avant du logement hôte (111) ;
un appareil de surveillance (201), lequel est assemblé avec la structure d'assemblage (120) et peut être désassemblé de la structure d'assemblage (120) ; où l'appareil de surveillance (201) peut mettre en œuvre une fonction de surveillance indépendamment du fait qu'il soit assemblé avec la structure d'assemblage (120) ou non, où l'hôte (101) est en liaison de communication avec l'appareil de surveillance (201) par le biais de l'unité de transmission d'informations hôte pour mettre en œuvre une transmission d'informations unidirectionnelle ou une transmission d'informations bidirectionnelle ;
un premier élément de stockage (301), lequel est configuré pour stocker un accessoire de surveillance, **caractérisé en ce que** le premier élément de stockage (301) est relié de manière amovible à l'appareil de surveillance (201), ou le premier élément de stockage (301) est fixé à l'appareil de surveillance (201), ou le premier élément de stockage (301) et l'appareil de surveillance (201) sont formés de manière solidaire ; où le premier élément de stockage (301) peut être transféré en même temps que l'accessoire de surveillance et l'appareil de surveillance (201).

2. Dispositif de défibrillation et de surveillance selon la revendication 1, **caractérisé en ce qu'**au moins deux surfaces du logement hôte définissent une zone d'assemblage pour module d'extension ; où au moins une partie de l'appareil de surveillance est stockée à l'intérieur de la zone d'assemblage pour module d'extension, lorsque l'appareil de surveillance est assemblé avec la structure d'assemblage.

3. Dispositif de défibrillation et de surveillance selon la revendication 1 ou 2, **caractérisé en ce que** l'appareil de surveillance est agencé au niveau d'au moins l'un : d'un côté gauche, d'un côté droit, d'un côté arrière, d'un côté supérieur et d'un côté inférieur de l'unité d'affichage hôte, lorsque l'appareil de surveillance est assemblé avec la structure d'assemblage ;
de préférence, l'appareil de surveillance est agencé au niveau du côté arrière de l'unité d'affichage hôte, lorsque l'appareil de surveillance est assemblé avec la structure d'assemblage ; ou l'appareil de surveillance peut être placé dans et/ou sorti d'une zone d'assemblage pour module d'extension, depuis au moins l'un : d'un côté arrière, d'un côté gauche, d'un côté droit, d'un côté supérieur et d'un côté inférieur de la zone d'assemblage pour module d'extension ; ou au moins l'un : d'un côté arrière, d'un côté gauche, d'un côté droit, d'un côté supérieur et d'un côté inférieur d'une zone d'assemblage pour module d'extension, est ouvert, de telle sorte que l'appareil de surveillance peut être placé dans et/ou sorti de la zone d'assemblage pour module d'extension, depuis le au moins un côté ouvert ; où le au moins un côté ouvert est exposé ou doté d'une structure de barrière qui peut être ouverte et fermée.

4. Dispositif de défibrillation et de surveillance selon la revendication 2, **caractérisé en ce que** le logement hôte est doté d'une plateforme de support, laquelle comprend une surface de support, où les au moins deux surfaces du logement hôte, lesquelles définissent la zone d'assemblage pour module d'extension, comprennent la surface de support ; où la zone d'assemblage pour module d'extension est agencée au niveau d'un côté supérieur de la plateforme de support, et la surface de support est configurée pour supporter l'appareil de surveillance ;
de préférence, la plateforme de support est agencée au niveau d'un côté arrière de l'unité d'affichage hôte, et l'appareil de surveillance peut être placé dans et/ou sorti de la plateforme de support, depuis au moins l'un ; d'un côté arrière, d'un côté gauche, d'un côté droit et d'un côté supérieur de la zone d'assemblage pour module d'extension ; ou un premier angle est formé entre l'unité d'affichage hôte et une direction verticale, et la surface de support est inclinée vers le bas suivant une direction avant-arrière pour le premier angle, lorsque le dispositif de défibrillation et de surveillance est positionné horizontalement.

5. Dispositif de défibrillation et de surveillance selon la revendication 2, **caractérisé en ce que** les au moins deux surfaces du logement hôte, lesquelles définissent la zone d'assemblage pour module d'extension, comprennent une surface latérale qui est située devant la zone d'assemblage pour module d'extension, où l'unité de transmission d'informations hôte comprend une partie d'accueil, laquelle est reliée à l'appareil de surveillance, où la partie d'accueil est agencée au niveau de la surface latérale qui est située devant la zone d'assemblage pour module d'extension.

6. Dispositif de défibrillation et de surveillance selon l'une quelconque des revendications 1 à 5, **caractérisé en ce que** l'hôte comprend en outre un circuit de détection de bioimpédance thoracique, lequel est configuré pour acquérir des données de détection de bioimpédance thoracique ; où l'unité de commande hôte est configurée pour recevoir les données de détection de bioimpédance thoracique, de sorte à commander au circuit de charge et au circuit de décharge de réaliser le processus de charge et de décharge du condensateur haute tension ; ou
le premier élément de stockage comprend de multiples sous-éléments de stockage distincts, lesquels sont formés de manière solidaire, reliés de manière amovible, ou fixés les uns aux autres.

7. Dispositif de défibrillation et de surveillance selon l'une quelconque des revendications 1 à 6, **caractérisé en ce que** l'appareil de surveillance comprend un module de surveillance ; où le module de surveillance comprend une unité de commande, laquelle est configurée pour recevoir et traiter des données de surveillance, une unité d'affichage, laquelle est configurée pour afficher les données de surveillance, et une unité de transmission d'informations ;
où l'unité de transmission d'informations du module de surveillance est en liaison de communication avec l'unité de transmission d'informations hôte pour mettre en œuvre une transmission d'informations unidirectionnelle ou une transmission d'informations bidirectionnelle.

8. Dispositif de défibrillation et de surveillance selon la revendication 7, **caractérisé en ce que** le module de surveillance est doté d'une première structure d'accueil laquelle est reliée de manière amovible à la structure d'assemblage ;
de préférence, le premier élément de stockage est relié de manière amovible au module de surveillance.

9. Dispositif de défibrillation et de surveillance selon la revendication 7, **caractérisé en ce que** l'appareil de surveillance comprend en outre un deuxième élément de stockage ; où le deuxième élément de stockage est doté d'une chambre de stockage, laquelle est configurée pour stocker le module de surveillance, et d'une seconde structure d'accueil laquelle est reliée de manière amovible à la structure d'assemblage.

10. Dispositif de défibrillation et de surveillance selon la revendication 9, **caractérisé en ce que**
l'unité d'affichage du module de surveillance n'est pas bloqué par le deuxième élément de stockage, lorsque le module de surveillance est stocké à l'intérieur de la chambre de stockage ; où le deuxième élément de stockage est doté d'une unité de transfert d'informations, où l'unité de transfert d'informations comprend une partie d'accueil pour le module de surveillance et une partie d'accueil hôte, où la partie d'accueil pour le module de surveillance est en liaison de communication avec l'unité de transmission d'informations du module de surveillance, et la partie d'accueil hôte est en liaison de communication avec l'unité de transmission d'informations hôte ; ou
le deuxième élément de stockage comprend de multiples sous-éléments de stockage distincts, lesquels sont formés de manière solidaire, reliés de manière amovible ou fixés les uns aux autres ; ou
le premier élément de stockage et le deuxième élément de stockage sont formés de manière solidaire, ou le premier élément de stockage est relié de manière amovible au second élément de stockage, ou le premier élément de stockage est fixé au deuxième élément de stockage.

11. Dispositif de défibrillation et de surveillance selon l'une quelconque des revendications 1 à 10, **caractérisé en ce que** le premier élément de stockage est agencé au niveau d'au moins l'un : d'un côté arrière, d'un côté gauche et d'un côté droit de l'appareil de surveillance ;
de préférence, les côtés gauche et droit d'un module de surveillance ou d'un deuxième élément de stockage sont tous deux dotés du premier élément de stockage.

12. Dispositif de défibrillation et de surveillance selon la revendication 1, **caractérisé en ce qu'**une projection du premier élément de stockage sur un plan de coupe transversale chevauche au moins en partie une projection de l'hôte sur ledit plan de coupe transversale, où ledit plan de coupe transversale est le long d'une direction avant-arrière et perpendiculaire à une surface inférieure du dispositif de défibrillation et de surveillance ; ou
une projection du premier élément de stockage sur un plan de coupe transversale chevauche en partie ou ne chevauche pas une projection de l'hôte sur ledit plan de coupe transversale, où ledit plan de coupe transversale est le long d'une direction gauche-droite et perpendiculaire à une surface inférieure du dispositif de défibrillation et de surveillance.

13. Dispositif de défibrillation et de surveillance selon l'une quelconque des revendications 1, 4 ou 5, **caractérisé en ce qu'**il comprend en outre un troisième élément de stockage, lequel est configuré pour stocker une ou des électrodes patchs et/ou un ou des câbles pour électrodes patchs ;
où le troisième élément de stockage est relié de manière amovible à l'appareil de surveillance ; ou le troisième élément de stockage est relié de manière amovible à l'hôte, ou le troisième élément de stockage est fixé à l'hôte, ou le troisième élément de stockage et l'hôte sont formés de manière solidaire ;
de préférence, le troisième élément de stockage est agencé au niveau d'une surface latérale qui est située devant une zone d'assemblage pour module d'extension et/ou au niveau d'un côté arrière d'une plateforme de support ; ou le troisième élément de stockage est parallèle à l'unité d'affichage hôte.
